# EUROPEAN PATENT APPLICATION

(11) **EP 4 666 938 A1**
(43) Date of publication of application: **24.12.2025**
(21) Application number: 24183824.2
(22) Date of filing: 21.06.2024
(51) Int. Cl.: A61B 5/0205, A61B 5/024, A61B 5/055, A61B 5/00, G16H 30/40, G16H 50/20, G16H 50/30, A61B 5/08, A61B 5/11

(54) **AUTO PATIENT PANIC DETECTION DURING MEDICAL IMAGING**

(71) Applicant: Siemens Healthineers AG, 91301 Forchheim (DE)
(72) Inventor: ARAVIND, Yarlagadda, 560100 Bangalore (IN)
(74) Representative: HKW Intellectual Property PartG mbB

(57) **Abstract**

The invention relates to a system for acquiring medical images of a patient comprising: a medical imaging unit for acquiring the medical images; at least one sensor for acquiring sensor data of the patient; an input unit for receiving the sensor data; an emergency level determination unit for deriving at least one value concerning at least one attribute of the patient based on the received sensor data, and for determining an emergency level of the patient based on the derived at least one value using an emergency level determination algorithm; and an output unit for informing a technician using the medical imaging unit about the determined emergency level and/or for influencing the usage of the medical imaging unit based on the determined emergency level.

In an advantageous manner, the system can automatically detect an emerging and/or existing panic attack of the patient.

## Description

The present invention relates to a system for acquiring medical images for providing auto panic detection, a method for using said system and a computer program product.

During a medical examination with the objective to take medical images of a patient, the patient is positioned within and/or in vicinity of a medical imaging unit. The medical imaging unit can be, in particular, an MRI (magnetic resonance imaging) scanner, an x-ray scanner, a mammography scanner, a computed tomography (CT) scanner, and/or the like. Hereby, the patient may be positioned on a bed of the medical imaging unit slowly moving through a gantry.

The medical imaging unit can produce a strong magnetic field that forces protons in the body to align with that field. A radiofrequency current can then be pulsed through the patient; thus, protons can be stimulated, and possibly spin out of equilibrium, straining against a pull of the magnetic field. Said protons are picked up by detectors and transmitted to a computer that processes the data into an image. The use of such medical images for patients has many benefits for medical diagnosis and analysis. Nonetheless, the gantry may be characterized as representing a narrow and long tunnel in which the patient is lying during the examination. Hereby, the medical imaging unit may fill the entire field of view of the patient due to the gantry's large dimensions. In addition, during the examination, the medical imaging unit generates significant noise. In consequence, the patient may experience strong emotions before and/or during the examination such as loss of control, fear, anxiety, and/or trauma. Potentially, the patient may even show physical symptoms like trembling and/or unconsciousness. Said is true especially in a case the patient is claustrophobic. In the following, said negative emotional and physical experiences are referred to as a panic attack. Note that in this context, the term "panic attack" has to be understood as any kind of emergency situation due to the physical and/or psychological condition of the patient. Independent of the grammatical term usage, individuals with male, female or other gender identities are included within the term.

In case a patient experiences a panic attack, the examination must be interfered, delayed, or even stopped to prevent significant psychological and/or physical damage of the patient. Currently there is no automated technology that can avoid and/or detect a patient experiencing a panic attack within and/or in vicinity of the medical imaging unit. Thus, prior to conducting the examination, an extensive pre-medical checkup is conducted to the patient to estimate the patient's condition and suitability for the examination. Yet, during the examination, it is up to the alertness of a technologist using the medical imaging unit or the reliance on a panic balloon to detect a panic attack. The panic balloon is a mechanical or electrical button the patient can activate for signaling a panic attack or other emergency to the technologist. Nonetheless, especially in case the patient is unconscious, said current technologies suffer in terms of their reliability and promptness.

Therefore, it is one objective of the present invention to improve the acquiring of medical images.

According to a first aspect, a system for acquiring medical images of a patient is suggested, the system comprising
a medical imaging unit for acquiring the medical images;
at least one sensor for acquiring sensor data of the patient;
an input unit for receiving the sensor data;
an emergency level determination unit for deriving at least one value concerning at least one attribute of the patient based on the received sensor data, and for determining an emergency level of the patient based on the derived at least one value using an emergency level determination algorithm; and
an output unit for informing a technician using the medical imaging unit about the determined emergency level and/or for influencing the usage of the medical imaging unit based on the determined emergency level.

The medical imaging unit can be an MRI Scanner, a CT scanner, an x-ray scanner, a mammography scanner, and/or another device configured for taking medical images. The medical imagining unit may comprise a gantry which can have a magnetic field within the gantry. The gantry may surround a tube, through which a bed can be moved. The bed can be located on top of a table. The bed can be configured to be movable with respect to an axial direction. The patient can be positioned on the bed in a lying manner.

The system further comprises at least one sensor for acquiring sensor data of the patient. Preferably, the system comprises a plurality of sensors. In particular, the at least one sensor can be a camera for capturing a face of the patient, a respiratory sensor for detecting a beathing condition of the patient, an ECG sensor for detecting heartbeats of the patient, a blood pressure sensor for detecting a blood pressure of the patient, and/or a kinetic sensor for detecting a movement of the patient. In this context, the term "sensor data" means signals from the at least one sensor. The at least one sensor is configured to acquire data of the patient, representing a physical and/or psychological condition of the patient. The at least one sensor can be attached to the patient and/or be in vicinity of the patient.

The system further comprises the input unit for receiving the sensor data. In particular, the input unit is configured to convert the sensor data into a computer-readable value or values. The input unit can be configured as a hardware interface for a computer device. In case the system comprises a plurality of sensors, the sensor data of each of the plurality of sensors is received by the input unit and converted into computer-readable values.

The system further comprises an emergency level determination unit. The emergency level determination unit is configured to derive at least one value concerning at least one attribute of the patient based on the received and converted sensor data. In this context, the term "attribute" has to be understood as a property and/or a behavior of the patient expressing the patient's psychological and/or physical condition. For example, an attribute can be an average heart rate, a regularity of the heart rate, a head movement, a breathing rate status, and/or an emotion. In the present context, the term "values" represents feasible data which can be addressed to the respective attribute. For example, the attribute "average heart rate" can express one of the values "normal", "stage 1 hypertension", "stage 2 hypertension", or "high hypertension". The attribute "regularity of the heart rate" can express one of the values "regular" or "irregular". The attribute "head movement" can express one of the values "stable" or "unstable". The attribute "breathing rate status" can express one of the values "normal", "hypoventilation" or "hyperventilation". The attribute "emotion" can express one of the values "fear", "anxiety", "emotionless", or "unconsciousness". Note that there can exist other attributes and/or other values to said attributes.

The emergency level determination unit is further configured to determine an emergency level of the patient based on the derived values using an emergency level determination algorithm. The emergency level represents an indicator for the possible emerging or existence of a panic attack of the patient. The emergency level determination unit can be designed using electronical components, in particular, using integrated circuits. Yet, preferably, the emergency level determination unit is designed as a computer program product. The emergency level determination algorithm is a model run by the emergency level determination unit. The emergency level determination algorithm can determine said emergency level based on selected ones of the derived attributes or based on all the derived attributes.

Based on the determined emergency level, the technician can be informed. It is feasible to inform the technician using an acoustical and/or visual alarm. Alternatively, or additionally, the system can be configured to automatically influence the usage of the medical imaging unit based on the determined emergency level. For example, in case the patient Is likely to experience a panic attack or already experiencing such, the examination can be influenced, delayed, and/or stopped by the system. In this context, to influence the usage of the medical imaging unit can mean, for example, to reduce the number of medical images during the examination, thus shorten the duration of the examination, and/or to provide the patient a visual and/or acoustic distraction and/or feedback concerning the upcoming examination.

In an advantageous manner, the system can automatically detect an emerging and/or existing panic attack of the patient. In consequence, a possible threat to the physical and/or psychological health of the patient can be reduced and/or avoided. Furthermore, due to the possibility to influence the usage of the medical imaging unit, the emerging of a panic attack can possibly be prevented.

According to an embodiment, the at least one sensor is a camera for capturing a face of the patient, a respiratory sensor for detecting a beathing condition of the patient, an ECG sensor for detecting heartbeats of the patient, a blood pressure sensor for detecting a blood pressure of the patient, and/or at least one kinetic sensor for detecting a movement of the patient, in particular, the at least one kinetic sensor is designed to detect a movement of a head, an arm, a leg, and/or other body parts of the patient.

The camera is compatible with the medical imaging unit. In particular, the camera is compatible with magnetic fields and/or x-ray radiation. In particular, it is free of interferences with the medical imaging unit. The camera is compact in size and lightweight and features a selectable focal length. The camara is capable of capturing single images and/or real-time videos. The ECG sensor can be integrated in the bed underneath the patient and/or be attached to a hand, a chest, and/or an arm of the patient. The location of the ECG sensor can be set according to the purpose of the acquiring of medical images. For example, in case the medical imaging unit is an MRT scanner, the ECG sensor can be located underneath the patient while in case the medical imaging unit is a CT scanner, the ECG scanner can be attached to the leg and/or the arm of the patient. The same applies to the blood pressure sensor. The at least one kinetic sensor can be used to recognize a movement of at least one body part of the patient, for example a movement of the head, of the arm, or of another body part. Preferably, the system comprises a plurality of kinetic sensors, for example two, three, four, or five. The usage of a plurality of kinetic sensors enables the detection of a movement of the patient in a three-dimensional manner. The system may comprise further sensors, for example sensors configured to measure a skin temperature of the patient, a humidity of the skin of the patient, or other properties and/or behaviors of the patient capable of expressing the physical and/or psychological condition of the patient.

According to a further embodiment, the emergency level determination unit is configured such that the at least one value concerning at least one of the two attributes "average heart rate" of the patient and "regularity of the heart rate" is derived based on the heartbeats detected by the ECG sensor.

The emergency level determination unit is configured such that it can derive the attribute "average heart rate" based on the heartbeats detected by the ECG sensor. The attribute "average heart rate" represents the average number of heartbeats occurring per minute. Alternatively, or additionally, the emergency level determination unit is configured to derive the attribute "regularity of the heart rate" based on the detected heartbeats. The attribute "regularity of the heart rate" expresses how regular the heartbeats of the patients occur. Its value "regular" in this context means that all durations between two subsequent heartbeats are substantially identical. In contrast, the value "irregular" means that said durations vary significantly. In an advantageous manner, to distinguish between the average heart rate and the regularity of the heart rate enhances the possibility to detect a panic attack of the patient.

According to a further embodiment, the at least one sensor is integrated within the medical imaging unit. In particular, the at least one sensor is installed above and facing the patient and/or the at least one sensor is integrated within a bed of the medical imaging unit.

The at least one sensor can be integrated in the gantry of the medical imaging unit and above the bed. In particular, if the at least one sensor is a camera or a kinetic sensor, said installation provides a beneficial field of view towards the patient. In particular, the head and/or the face of the patient are detectable by the at least one sensor. Additionally, or alternatively, the at least one sensor can be integrated within the bed of the medical imaging unit. This way, the patient can be positioned on top of the at least one sensor, enabling a convenient usage while securing a reliable data acquisition. Other locations for the at least one sensor, for example, on top of the bed, are feasible as well, for example, in case the at least one sensor is an ECG sensor or a blood pressure sensor. In a beneficial manner, this way, the at least one sensor is not exposed to the patient or the technician, thus significantly reducing the risk of accidentally detaching and/or damaging the at least one sensor.

According to a further embodiment, the system further comprises a facial expression determination unit for providing the at least one value of the at least one attribute by determining a facial expression of the patient using a facial expression determination algorithm. In particular, the at least one value represents an emotion and/or a physical condition of the patient.

The facial expression determination unit is configured to provide at least the attribute "emotion" of the patient. In addition, the facial expression determination unit can also provide an attribute representing whether the patient is conscious or unconscious. The facial expression determination unit may provide other attributes as well. Therefore, the input unit receives and converts the sensor data of the camera providing a footage of the patient's face. The facial expression determination unit is configured to utilize the converted data in order to capture a facial expression of the patient. The facial expression determination unit assigns a plurality of markers to features within the footage of the face of the patient, enabling a measurement of the facial expression of the patient. For example, the markers can represent the locations of eyes, nose, mouth, and/or chin of the patient. A plurality of markers can be arranged along lips of the patient, enabling a mathematical description of the size and the geometrical form of a possibly opened mouth. According to internal knowledge of the applicant, the facial expression of the patient, precisely localized by the locations of the markers, is representative of the emotional condition of the patient. Furthermore, the facial expression of the patient represents physical conditions of the patient, for example, whether the patient is conscious or unconscious. Utilizing the locations of the markers, the facial expression determination unit is configured to determine the attribute "emotion" and/or other attributes by executing the facial expression determination algorithm. The facial expression determination unit can be configured as a computer program product. The facial expression determination algorithm can be configured as a model.

According to a further embodiment, the facial expression determination algorithm is a machine learning algorithm and/or a map-based algorithm, in particular, the facial expression determination algorithm is a classifier.

The facial expression determination algorithm can be a deep learning algorithm. The facial expression determination algorithm is configured to classify the locations of the markers within the footage of the face of the patient into values of the attribute "emotion". Each value represents one possible emotion of the patient, for example fear, anxiety, emotionless, or others. During a learning phase of the facial expression determination algorithm, said emotion values are assigned to a large number of footages of patients' faces by human annotators who have been trained to accurately identify emotional expressions. That is used as training data of the facial expression determination algorithm. Hereby, the training data set is diverse and representative of a wide range of individuals and different ethnicities ensuring that the trained facial expression determination algorithm is robust and capable of correctly identifying the emotion of patients over a wide range of different ethnicities and age groups.

According to a further embodiment, the emergency level of the patient is a binary information representing whether a panic attack is occurring or not and/or a numerical value representing how probable a panic attack is likely to occur.

In this context, the term "numerical value" may be understood as a float number. In case the emergency level is a numerical value, the emergency level can be any numerical value within a specific range while the range is set by two numerical values representing a minimum and a maximum probability for the existence of a panic attack. The usage of a numerical value as emergency level enables a more precise evaluation of the condition of the patient. A low number can express that the patient is experiencing only slight indications of a panic attack. A high number can express that the patient is close to a panic attack or already experiencing a panic attack. The numerical emergency level enables a precise influencing of the medical imaging unit according to the current condition of the patient. Thus, potentially, a further emerging of the emergency level can be prevented.

According to a further embodiment, the emergency level determination algorithm is a machine learning algorithm and/or a map-based algorithm, in particular, the emergency level determination algorithm is a classifier.

In particular, the emergency level determination algorithm can be a deep learning algorithm or be the result of the execution of a deep learning algorithm. The emergency level determination algorithm can be configured to classify the received value or values of the at least one attribute into the emergency level. Hence, the emergency level determination algorithm can be configured to determine the emergency level. Said determination of the emergency level can occur automatically, promptly and/or in real-time based on the received sensor data. This enables a prompt identification of a potential panic attack and enables instant measures accordingly.

According to a further embodiment, the emergency level determination algorithm is configured such that during a learning phase, the emergency level determination algorithm is trained using supervised learning, wherein the derived values concerning the attributes are used as training data, and wherein the learning phase results in the creation of a decision tree.

During a learning phase of the emergency level determination algorithm, said emergency levels are assigned to a large number of datasets of training data, each including the value or values of the at least one attribute, by human annotators who have been trained to accurately identify the corresponding emergency levels. This way, the emergency level determination algorithm is trained in the manner of supervised learning. Hereby, the training data set is diverse and representative of a wide range of individuals ensuring that the trained emergency level determination algorithm is robust and capable of correctly identifying the emergency level of patients over a wide range of different ethnicities and age groups. Additionally, or alternatively, the emergency level determination algorithm can be trained using unsupervised learning, as well. The result of the learning phase is the creation of at least one decision tree. The at least one decision tree comprises a plurality of child nodes, wherein each child node represents one of the attributes. The number of branches of each of the child nodes represents the number of available values concerning the respective attribute. Each branch of the decision tree ends in a leaf node, representing the emergency level of the patient based on the current values of the attributes. In a beneficial manner, the created decision tree can accelerate the determination of the emergency level of the patient during the usage of the system. In addition, the determination of the emergency level can be conducted in a reliable manner.

According to a further embodiment, the emergency level determination algorithm is configured such that, during the learning phase, specific ones of the attributes are chosen that best split the training data into distinct classes. In particular, specific ones of the attributes are chosen according to a measure of information gain of the attributes, in particular, measures of information gain such as entropy or Gini index are applied.

The attributes, for example "emotion", "average heart rate", "head movement", "breathing rate status", or others, are used as training data for the emergency level determination algorithm. Yet not all of the attributes may represent a similar information gain in determining the emergency level. It is an objective of the learning phase to best split the data set with the different attributes into distinct classes providing a maximized amount of information gain. Hence, during the learning phase, specific ones of the attributes are chosen according to a measure of information gain regarding each of the attributes. Different manners can be used to determine the information gain of each of the attributes. Possible manners are statistical methods like entropy or Gini index. The reduction to selected attributes can accelerate the determination of the emergency level during usage of the system.

According to a further embodiment, the emergency level determination algorithm is configured such that, during the learning phase, for each value of the chosen attribute, a child node is created.

According to a further embodiment, the emergency level determination algorithm is configured such that, during the learning phase, a random forest procedure is applied.

During the learning phase of the emergency level determination algorithm, all data sets of the training data can be used in order to create a single decision tree. Additionally, or alternatively, the random forest procedure can be applied. In this context, the random forest procedure means that a large number of decision trees is generated, each decision tree being created during an individual learning phase and based on an individual subset of all the training data. In other words, the whole set of training data is split into a variety of subsets, whereby each of the subsets is used to create an individual decision tree. After the creation of the decision trees, they are averaged to form a single final decision tree. In a beneficial manner, the hereby created averaged decision tree is characterized by a high predictive accuracy and a lower tendency of overfitting.

According to a further embodiment, the emergency level determination algorithm is configured such that it utilizes the created decision tree on the derived values based on the sensor data of the patient to determine the emergency level of the patient.

The decision tree is configured to classify the values of the attributes into the binary information and/or numerical value of the emergency level. In other words, the output of the decision tree is the binary information and/or the numerical value of the emergency level.

According to a second aspect, a method for using a system with a medical imaging unit for acquiring medical images of a patient is proposed. The method comprises
acquiring sensor data of the patient by at least one sensor;
receiving the sensor data by an input unit;
deriving at least one value concerning at least one attribute based on the received sensor data by an emergency level determination unit;
training an emergency level determination algorithm by the emergency level determination unit using the derived at least one value;
determining an emergency level of the patient by the emergency level determination unit based on the derived at least one value concerning the at least one attribute using the trained emergency level determination algorithm; and
informing a technician using the medical imaging unit about the determined emergency level of the patient and/or influencing the usage of the medical imaging unit based on the determined emergency level of the patient by an output unit.

The system is embodied according to the first aspect or according to an embodiment of the first aspect.

According to a third aspect, a computer program product is proposed comprising instructions which, when the program is executed by a computer, cause the computer to carry out the above-mentioned method.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the system of the first aspect apply mutatis mutandis to the method of the second aspect.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematic perspective illustration of an embodiment of a system for acquiring medical images of a patient;
Fig. 2 shows a schematic illustration of a working principle of an embodiment of a facial expression determination unit of the system according to Fig. 1;
Fig. 3 shows a schematic illustration of a decision tree as created by an embodiment of an emergency level determination algorithm of the system according to Fig. 1;
Figs. 4 and 5 show two samples of sensor data of an embodiment of an ECG sensor of the system according to Fig. 1;
Fig. 6 shows an embodiment of a method for using the system according to Fig. 1; and
Fig. 7 shows a schematic perspective illustration of a further embodiment of the system for acquiring medical images of the patient.

In the Figures, like reference numerals designate like or functionally equivalent elements, unless otherwise indicated.

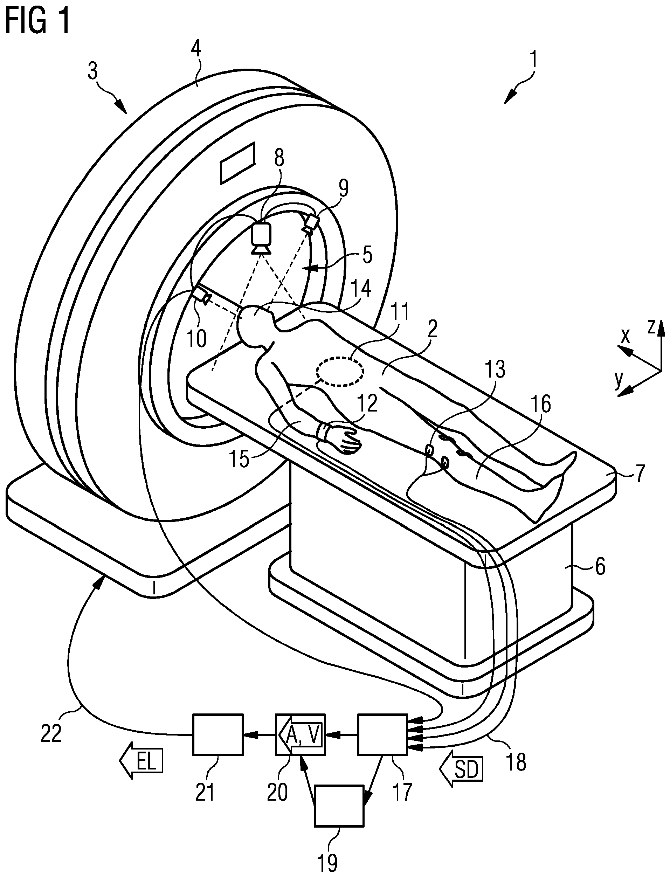

Fig. 1 shows a schematic perspective illustration of an embodiment of a system 1 for acquiring medical images of a patient 2. The system 1 comprises a medical imaging unit 3 with a gantry 4, whereby the gantry 4 contains magnetic fields (not visible in Fig. 1). The gantry 4 surrounds a tube 5 in the shape of a narrow and long tunnel. The tube 5 extends along an axial direction x. The medical imaging unit 3 further comprises a table 6 onto which a bed 7 is attached. The bed 7 is attached to the table 6 such way that the bed 7 can be moved along the axial direction x. In particular, the bed 7 can be moved through the gantry 4. Hereby, the tube 5 can possibly fill the entire field of view of the patient 2.

In consequence, the patient 2 may experience strong emotions before and/or during an examination with the medical imaging unit 3 such as loss of control, fear, anxiety, and/or trauma. Potentially, the patient 2 may even show physical symptoms like trembling and/or unconsciousness. Said is true especially in case the patient 2 is claustrophobic. In case the patient 2 experiences a panic attack, the examination must be interfered, delayed, or even stopped to prevent significant psychological and/or physical damage of the patient 2.

The system 1 further comprises a plurality of sensors 8 - 13, in particular, it comprises a camera 8, two kinetic sensors 9, 10, a respiratory sensor 11, a blood pressure sensor 12, and an ECG sensor 13. The camera 8 is integrated within the gantry 4 and on top of the bed 7 facing downwards. This way, the camera 8 is configured to capture a head and/or a face 14 of the patient 2. The kinetic sensors 9, 10 are integrated within the gantry 4 as well at different locations inside the tube 5. This way, the kinetic sensors 9, 10 are configured to capture the head and/or the face 14 from different angles and thus enable capturing the head and/or the face 14 in a three-dimensional manner. The respiratory sensor 11 is integrated in the bed 7 underneath the patient 2. This way, the breathing status of the patient 2 can be detected by the respiratory sensor 11. In particular, the respiratory sensor 11 is configured to detect a diaphragm state of the patient 2. The blood pressure sensor 12 is attached to an arm 15 of the patient 2 for detecting a blood pressure of the patient 2. The ECG sensor 13 is attached to a leg 16 of the patient 2 for detecting heartbeats H of the patient 2. The ECG sensor 13 can be attached to the arm 15 of the patient 2, as well.

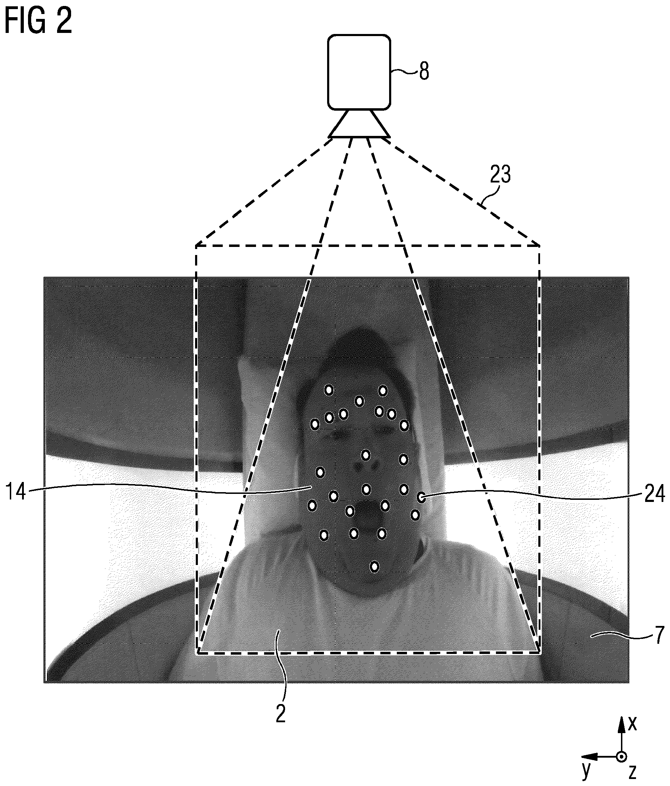

The sensors 8 - 13 are coupled with an input unit 17 via at least one sensor cable 18 of which only one is referred to with a reference sign in Fig. 1 Alternatively, or additionally, the sensors 8 - 13 can be coupled with the input unit 17 in a wireless manner. The input unit 17 converts sensor data SD of the sensor 8 - 13 into computer-readable digital data. A facial recognition determination unit 19 is configured to determine an emotion of the patient 2 and will be described in detail with reference to Fig. 2.

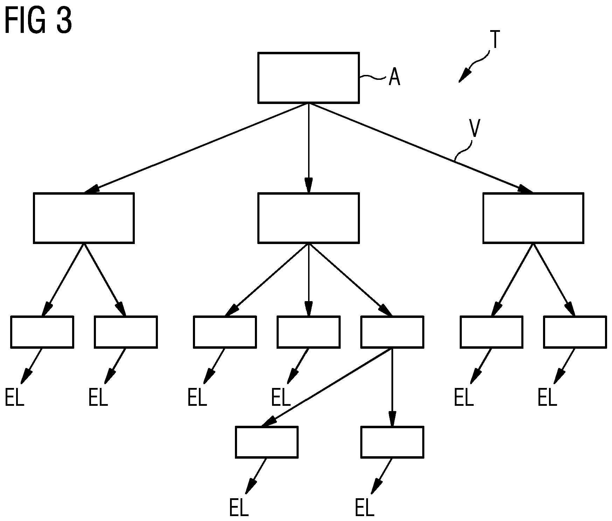

The system 1 further comprises an emergency level determination unit 20 which is configured to derive a plurality of values V concerning a plurality of attributes A based on the sensor data SD. For example, the attributes A can be an average heart rate, a regularity of the heart rate, a head movement, and/or a breathing rate status. Said attributes A can also be referred to a physiological indicators. In the present context, the values V represent feasible data which can be addressed to the respective attributes A. For example, the attribute A "average heart rate" can express one of the values V "normal", "stage 1 hypertension", "stage 2 hypertension", or "high hypertension". The attribute A "regularity of the heart rate" can express one of the values V "regular" or "irregular". The attribute A "head movement" can express one of the values V "stable" or "unstable". The attribute A "breathing rate status" can express one of the values V "normal", "hypoventilation" or "hyperventilation". Note that there can exist other attributes and/or other values V to said attributes A. Furthermore, the emergency level determination unit 20 is configured to determine an emergency level EL of the patient 2 based on the derived values V. The emergency level EL can be a binary information expressing whether the patient 2 is likely to experience and/or is already experiencing a panic attack. Additionally, or alternatively, the emergency level EL can be a numerical number in the sense of a float number expressing the probability of an emerging of a panic attack of the patient 2. The determination of the emergency level EL will be described in detail with reference to Fig. 3.

The system 1 further comprises an output unit 21 which is configured to inform a technician (not visible in the figures) using the medical imaging unit 3 about the determined emergency level EL, possibly by an acoustic and/or visual indication. Hence, the output unit 21 can inform the technician in case the patient 2 experiences a panic attack or is likely to experience such. In addition, or alternatively, the output unit 21 is configured to convert the determined emergency level EL into an electrical and/or electronical signal which can be transferred to the medical imaging unit 3 via an output cable 22, for example. The medical imaging unit 3 can be influenced based on the determined emergency level EL, for example to delay, or to stop the examination of the patient 2.

The facial recognition determination unit 19 and/or the emergency level determination unit 20 can be part of a software for controlling the medical imaging unit 3.

Fig. 2 shows a schematic illustration of a working principle of an embodiment of the facial expression determination unit 19. During the examination, the patient 2 is positioned on the bed 7 in such a way, that the head and/or the face 14 is within a field of view 23 of the camera 8. This way, the camera 8 is configured to capture a footage of the head and/or the face 14. In Fig. 2, an exemplary footage of the head and/or the face 14 of a patient 2 is illustrated as captured by the camera 8. A plurality of markers 24 are assigned to the footage by the facial expression determination unit 19 of which only one is assigned with a reference number in Fig. 2. The markers 24 are allocated to locations of significant facial elements, representing a facial expression of the patient 2. For example, the markers 24 can capture whether the mouth of the patient 2 is open (Fig. 2). The locations of the markers 24 are used as input data for a deep neural network, in the following referred to as the facial expression determination algorithm, which is executed by the facial expression determination unit 19. The facial expression determination algorithm is a classifier, classifying the locations of the markers 24 into specific values V of the attribute A "emotion". The attribute A "emotion" can express one of the values V "fear", "anxiety", "emotionless", "unconsciousness", or others, and can be referred to as vital indicator as well. During a training phase of the facial expression determination algorithm, a large number of footages of patients 2 showing different emotions are complemented by the value V corresponding to the respective emotions, which is assigned by specially trained human annotators. Hereby the footages cover a wide range of different ethnicities and/or age groups. Said data are used as training data to train the facial expression determination algorithm. This way, the trained facial expression determination algorithm is capable of accurately identifying a human emotion according to the facial expression.

Fig. 3 shows a schematic illustration of a decision tree T as created by the emergency level determination algorithm. The emergency level determination algorithm is a deep neural network, In particular, a classifier, which is executed by the emergency level determination unit 20 and is configured to classify the values V of the attributes A into the emergency level EL. During a training phase in the sense of supervised learning, the emergency level determination algorithm is trained using the attributes A and their respective values V in combination with the corresponding emergency level EL which is assigned by specially trained human annotators. Hence, the combination of the number of values V concerning the plurality of attributes A and the corresponding emergency level EL form training data for the emergency level determination algorithm. For the training of the emergency level determination algorithm, subsets of all training data sets are used to create a large number of decision trees T of which only one is illustrated schematically in Fig. 3. The nodes of each decision tree T represent the different attributes A, whereby each node features a number of branches corresponding to the number of possible values V of the respective attribute A. During the training phase, the emergency level determination algorithm chooses specific attributes A among all available attributes A which split the training data best into distinct classes. The objective is to achieve a maximized information gain. The attributes A are selected using a measure of information gain, such as entropy and/or Gini index. By selecting the attributes A with the highest information gain, the trained decision trees T are characterized by a reduced complexity and, regarding a later execution of the emergency level determination algorithm during usage of the system 1, the accuracy and the computational speed of the emergency level determination algorithm are improved. Each branch ends in a leaf node, representing the corresponding emergency level EL. After creating the large number of decision trees T using the different subsets of the training data, all decision trees T are averaged to form an averaged single decision tree T. This procedure is referred to as a random forest procedure and can improve the predictive accuracy of the trained emergency level determination algorithm. Note, that the decision tree T illustrated in Fig. 3 is representable for the averaged single decision tree T, as well.

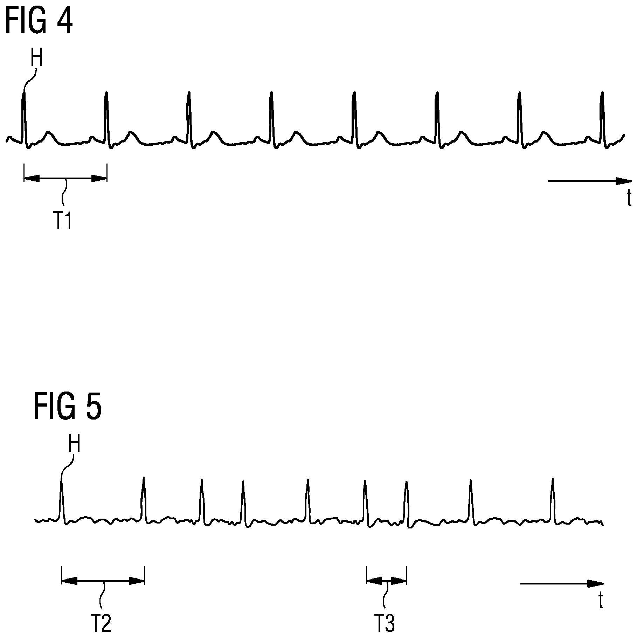

Figs. 4 and 5 show two samples of the sensor data SD of an embodiment of the ECG sensor 13 over time t, representing heartbeat schemes of two different patients 2 or a single patient 2 experiencing two different hear rate schemes. In the following, Figs. 4 and 5 will be referred to at the same time. The ECG sensor 13 is capable of detecting an activity of a heart of the patient 2, in particular, the ECG sensor 13 is configured to detect heartbeats H of the patient 2. The sensor data SD of the ECG sensor 13 show each heartbeat H as a distinct peak (Figs. 4, 5). In Figs. 4 and 5, only one heartbeat H is referred to with a reference number. The emergency level determination unit 20 is configured to use the sensor data SD of the ECG sensor 13 to calculate the attribute A "average heart rate" by averaging time durations T1, T2, T3 between two subsequent heartbeats H. In addition, the emergency level determination unit 20 is configured to use the sensor data SD of the ECG sensor 13 to derive the attribute A "regularity of the heart rate" by evaluating the regularity of the time durations T1, T2, T3. In case the time durations T1, T2, T3 between two subsequent heartbeats H are substantially constant, the value V "regular" is addressed to the attribute A "regularity of the heart rate". Such a regular heart rate is shown in an exemplary manner in Fig. 4. In contrast, in case the time durations T1, T2, T3 between two subsequent heartbeats H are diverting from one another in a significant manner, the value V "irregular" is addressed to the attribute A "regularity of the heart rate". Such an irregular heart rate is shown in an exemplary manner in Fig. 5.

In addition, based on the average heart rate, the emergency level determination unit 20 assigns different values V to the attribute A "average heart rate" describing the heart rate status, like, for example, "healthy", "elevated", "stage 1 hypertension", "stage 2 hypertension", "hypertension crisis" or others. Hereby, the detected blood pressure of the blood pressure sensor 12 is used to assign the value V of the attribute A "average heart rate" in a more precise manner.

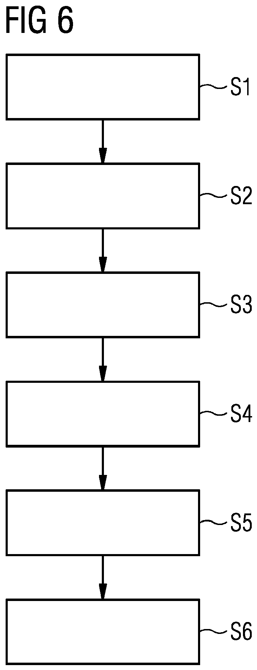

Fig. 6 shows an embodiment of a method for using the system 1 as described above. In a first step S 1, the sensor data SD of the patient 2 are acquired by the at least one sensor 8 - 13. In a second step S2, the sensor data SD are received by an input unit 17. Afterwards, in a third step S3, the at least one value V concerning the at least one attribute A is derived by the emergency level determination unit 20 based on the received sensor data SD. During a training phase S4, an emergency level determination algorithm is trained by using the derived at least one value V. In a fifth step S5, the emergency level determination unit 20 uses the trained emergency level determination algorithm to determine the emergency level EL of the patient 2 based on the derived at least one value V concerning the at least one attribute A. Finally, in a sixth step S6, the technician using the medical imaging unit 3 is informed about the determined emergency level EL and/or the usage of the medical imagining unit 3 is influenced. For example, the examination using the medical imaging unit 3 is delayed, interrupted, or stopped.

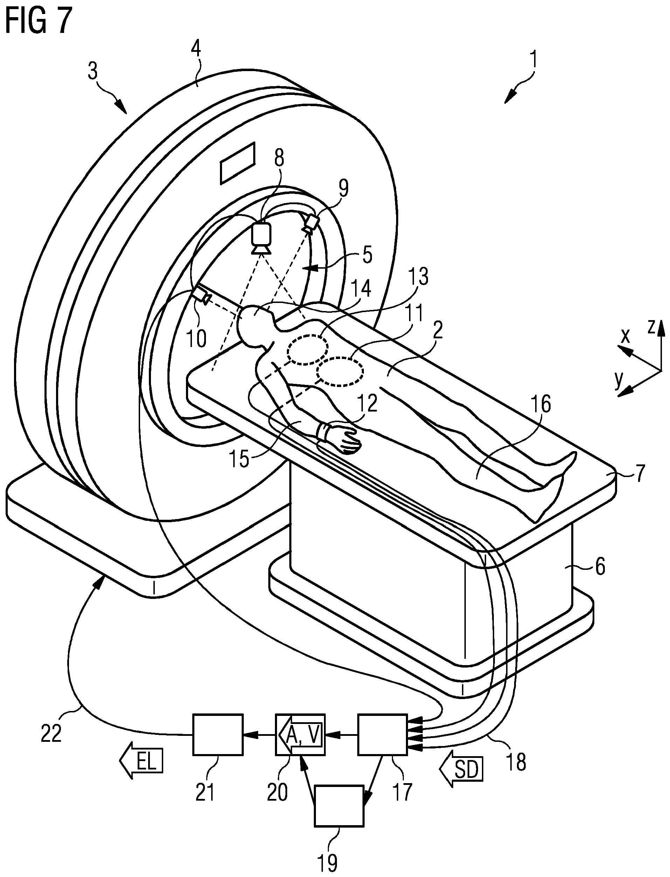

Fig. 7 shows a schematic perspective illustration of a further embodiment of the system 1 for acquiring medical images of the patient 2. Concerning that embodiment, the description of the above-mentioned embodiment is valid, as well. Hence, in the following, only the difference between both embodiments is addressed.

In the further embodiment of the system 1 according to Fig. 7, the ECG sensor 13 is integrated in the bed 7. Hence, with the patient 2 being placed lying on the bed 7, both the respiratory sensor 11 and the ECG sensor 13 are located underneath the patient 2. Hereby, the ECG sensor 13 can be located underneath the chest of the patient 2.

Although the present invention has been described in accordance with preferred embodiments, it is obvious for the person skilled in the art that modifications are possible in all embodiments.

## Claims

1. A system (1) for acquiring medical images of a patient (2), the system (1) comprising:
a medical imaging unit (3) for acquiring the medical images;
at least one sensor (8 - 13) for acquiring sensor data (SD) of the patient (2);
an input unit (17) for receiving the sensor data (SD);
an emergency level determination unit (20) for deriving at least one value (V) concerning at least one attribute (A) of the patient (2) based on the received sensor data (SD), and for determining an emergency level (EL) of the patient (2) based on the derived at least one value (V) using an emergency level determination algorithm; and
an output unit (21) for informing a technician using the medical imaging unit (3) about the determined emergency level (EL) and/or for influencing the usage of the medical imaging unit (3) based on the determined emergency level (EL).

2. The system according to claim 1, wherein the at least one sensor (8 - 13) is a camera (8) for capturing a face of the patient (2), a respiratory sensor (11) for detecting a beathing condition of the patient (2), an ECG sensor (13) for detecting heartbeats (H) of the patient (2), a blood pressure sensor (12) for detecting a blood pressure of the patient (2), and/or at least one kinetic sensor (9, 10) for detecting a movement of the patient (2), in particular, the at least one kinetic sensor (9, 10) is designed to detect a movement of a head (14), an arm (15), a leg (16), and/or other body parts of the patient (2).

3. The system according to claim 2, wherein the emergency level determination unit (20) is configured such that the at least one value (V) concerning at least one of the two attributes (A) describing an average heart rate of the patient (2) and a regularity of the heart rate is derived based on the heartbeats (H) detected by the ECG sensor (13).

4. The system according to one of claims 1 to 3, wherein the at least one sensor (8 - 13) is integrated within the medical imaging unit (3), particularly, the at least one sensor (8 - 13) is installed above and facing the patient (2) and/or the at least one sensor (8 - 13) is integrated within a bed (7) of the medical imaging unit (3).

5. The system according to one of claims 1 to 4, further comprising a facial expression determination unit (19) for providing the at least one value (V) of the at least one attribute (A) by determining a facial expression of the patient (2) using a facial expression determination algorithm; in particular, the at least one value (V) represents an emotion and/or a physical condition of the patient (2).

6. The system according to claim 5, wherein the facial expression determination algorithm is a machine learning algorithm and/or a map-based algorithm, in particular, the facial expression determination algorithm is a classifier.

7. The system according to one of claims 1 to 6, wherein the emergency level (EL) of the patient (2) is a binary information representing whether a panic attack is occurring or not and/or a numerical value representing how probable a panic attack is likely to occur.

8. The system according to one of claims 1 to 7, wherein the emergency level determination algorithm is a machine learning algorithm and/or a map-based algorithm, in particular, the emergency level determination algorithm is a classifier.

9. The system according to claim 8, wherein the emergency level determination algorithm is configured such that during a learning phase, the emergency level determination algorithm is trained using supervised learning, wherein the derived values (V) concerning the attributes (A) are used as training data, and wherein the learning phase results in the creation of a decision tree (T).

10. The system according to claim 9, wherein the emergency level determination algorithm is configured such that, during the learning phase, specific ones of the attributes (A) are chosen that best split the training data into distinct classes, in particular, specific ones of the attributes (A) are chosen according to a measure of information gain of the attributes (A), in particular, measures of information gain such as entropy or Gini index are applied.

11. The system according to claim 10, wherein the emergency level determination algorithm is configured such that, during the learning phase, for each value (V) of the chosen attribute (A), a child node is created.

12. The system according to one of claims 9 to 11, wherein the emergency level determination algorithm is configured such that, during the learning phase, a random forest procedure is applied.

13. The system according to one of claims 9 to 12, wherein the emergency level determination algorithm is configured such that it utilizes the created decision tree (T) on the derived values (V) based on the sensor data (SD) of the patient (2) to determine the emergency level (EL) of the patient (2).

14. A method for using a system (1) with a medical imaging unit (3) for acquiring medical images of a patient (2), the method comprising:
acquiring (S 1) sensor data (SD) of the patient (2) by at least one sensor (8 - 13);
receiving (S2) the sensor data (SD) by an input unit (17);
deriving (S3) at least one value (V) concerning at least one attribute (A) based on the received sensor data (SD) by an emergency level determination unit (20);
training (S4) an emergency level determination algorithm by the emergency level determination unit (20) using the derived at least one value (V);
determining (S5) an emergency level (EL) of the patient (2) by the emergency level determination unit (20) based on the derived at least one value (V) concerning the at least one attribute (A) using the trained emergency level determination algorithm; and
informing (S6) a technician using the medical imaging unit (3) about the determined emergency level (EL) of the patient (2) and/or influencing the usage of the medical imaging unit (3) based on the determined emergency level (EL) of the patient (2) by an output unit (21).

15. A computer program product comprising instructions which, when the program is executed by a computer, cause the computer to carry out the method of claim 14.
